# EUROPEAN PATENT SPECIFICATION

(11) **EP 0 321 020 B1**
(45) Date of publication and mention of the grant of the patent: **02.06.1993**
(21) Application number: 88202770.9
(22) Date of filing: 02.12.1988
(51) Int. Cl.: C07C 231/08, C07C 233/07

(54) **Process for preparing n-acyl-anilines**
Verfahren zur Herstellung von N-acyl-Anilinen
Procédé pour la préparation d'anilines N-acylés

(30) Priority: 16.12.1987 IT 2303587
(43) Date of publication of application: 21.06.1989
(73) Proprietor: ENIRICERCHE S.p.A., 20121 Milano (IT)
(72) Inventor: Clerici, Mario Gabriele, I-20097 San Donato Milanese Milan (IT); Di Carlo, Sabatino, I-20017 Rho Milan (IT); Belussi, Giuseppe, I-29100 Piacenza (IT)
(74) Representative: De Carli, Erberto

(56) References cited:
- No relevant documents disclosed

## Description

The present invention relates to a process for preparing N-acyl-anilines and, in particular, a process for preparing N-acetyl-para-amino-phenol.

N-acyl -anilines are useful products in the art, and in particular N-acetyl-para-amino-phenol, known under the name of "para-acetamol", is a well-known pharmaceutical component in the formulation of analgaesics, antipyretics and anti-inflammatories.

This compound performs a combined activity as an antibiotic, antiallergic and anti-inflammatory agent in many therapeutical compositions.

In the art various processes are known for preparing N-acetyl-para-amino-phenol. The most commonly used process uses the reaction between acetic anhydride and para-amino-phenol; this latter can be obtained by means of the reduction of para-nitro-phenol or of para-nitroso-phenol, or by hydrogenation of nitrobenzene under acidic conditions.

Other known processes are based on the Beckmann's rearrangement of para-hydroxy-acetophenone oxime, on the hydroxylation of acetanilide, and on the photolysis and pyrolysis of phenyl-hydrazide in acetic acid.

The present Applicant has found now that N-acyl-anilines can be prepared in a simple and cheap way, by making a phenol and an amide react with each other in the presence of a catalyst.

In accordance therewith, the present invention relates to a process for preparing N-acyl-anilines, with said process being characterized in that a phenol and an amide are made to react with each other by operating at a temperature within the range of from 200°C to 350°C and in the presence of a molecular sieve as the catalyst.

The reaction can be represented with the equation: 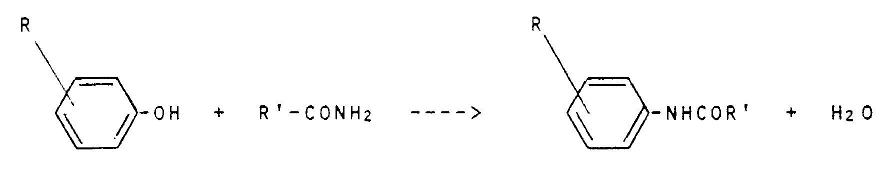
wherein:
- R: represents hydrogen, a lower alkyl (in particular methyl), a lower alkoxy (in particular, a methoxy), a halogen (in particular, chlorine), or hydroxy; and
- R′: represents an alkyl (in particular, a methyl), or an aryl (in particular, a phenyl).

According to a preferred form of practical embodiment, the present invention relates to a process for preparing N-acetyl-para-amino-phenol, with said process being characterized in that hydroquinone and acetamide are made to react with each other at a temperature within the range of from 200°C to 350°C, and in the presence of a molecular sieve as the catalyst.

By "molecular sieve", a crystalline aluminosilicate of a metal belonging to the Groups IA and IIA of the periodic system of the elements, and in particular of sodium, potassium, magnesium, and calcium is herein meant. For the purposes of the present invention, among the aluminosilicates, those having a high silica content are preferred. A typical example of an aluminosilicate useful for the intended purpose is the synthetic aluminosilicate known in the art under the name of ZMS-5, disclosed in US-P-3,702,882; and in Nature, 272, 437 (1978). These aluminosilicates are preferably used in their acidic form, i.e., free, or substantially free from metals belonging to Groups IA and IIA. This acidic form can be obtained, e.g., by submitting the aluminosilicate to an exchange with an ammonium salt, such as ammonium acetate, and submitting the so-exchanged product to a high-temperature heating, in order to remove ammonia.

By the term "molecular sieve", according to the present invention, also those products are meant, which are known in the art under the name of "silicalites".

The silicalites are topologically similar to ZSM-5, and can additionally contain in their structure substituting atoms of titanium, gallium, boron and iron. Examples of such substituted silicalites are reported, e.g., in EP-A-266825 and EP-A-265017 filed on October 19, 1987.

The molecular sieves, as catalysts, are prevailingly used in the art in petrochemical reactions, such as cracking reactions, polymerization, alkylation, isomerization, disproportioning, and the like.

Furthermore, in the technical literature also other reactions are reported, which take place under the influence of the molecular sieves, such as, e.g., the reactions of substitution of halogenated groups linked to an aromatic ring, the trans-halogenation reactions, the preparation of alpha-picolines by means of the treatment of phenol with ammonia or amines, and the synthesis of aromatic amines.

According to the preferred form of practical embodiment of the present invention, the molecular sieves, as hereinabove defined, are active in the reaction between hydroquinone and acetamide, and make it possible N-acetyl-para-amino-phenol to be produced, with a good yield, according to the equation

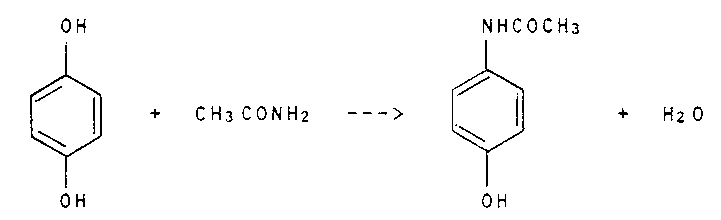

In this reaction, the molar ratio of hydroquinone to acetamide can be generally comprised within the range of from 1:0.5 to 1:20, but it will preferably be comprised within the range of from 1:1.5 to 1:10, in that improved results are obtained when the process is carried out with an excess of acetamide over the stoichiometric value.

The reaction temperatures are within the range of from 200 to 350°C, with preferred values being of the order of 250-300°C.

The reaction is furthermore advantageously carried out in the liquid phase, and in the absence, or in the presence, of a high-boiling solvent or diluent.

The addition of water to the reaction mixture is unfavourable for the equilibrium of the same reaction.

The reaction can be carried out either continuously or batchwise. In the second case, an amount of catalyst is advantageously used, which is comprised within the range of from 5 to 30 parts by weight per each 100 parts by weight of hydroquinone and acetamide reactants.

The reaction times are generally comprised within the range of from 0.5 to 3 hours, and the reaction mixture obtained can be treated by means of the usual methods for the separation and recovery of the reaction products.

The following experimental examples are reported in order to illustrate the invention in greater detail.

### Example 1

To a Carius tube (length 145 mm; diameter 23 mm), there are charged hydroquinone (1.00 g) and acetamide (0.78 g) in a molar ratio to each other of 1:1.6, together with the catalyst, as a powder, in an amount of 0.38 g. The catalyst used is ZMS-5 zeolite, having an Si/Al atomic weight of 93:3, transformed into the acidic form by treatment with ammonium acetate and subsequent heating at a high temperature.

The reaction tube is purged with a nitrogen stream, is evacuated (pressure lower than 1 mmHg), and is sealed by flame-welding. The Carius tube is then placed for 1 hour inside a cylindrical oven, the heating of which is controlled by means of a thermoregulator, and is heated to 300°C. After cooling and opening the tube, its contents are taken up with 25 ml of methanol.

The catalyst is centrifuged off, and the resulting solution is submitted to a gas-chromatographic quantitative analysis. The identity of the product is confirmed by mass-spectrometry, by being compared to authentic samples.

A conversion of hydroquinone of 93.6%, with a selectivity to N-acetyl-para-amino-phenol of 45.9 mol %, is determined.

### Examples 2-4

In these examples, the reaction between hydroquinone and acetamide is carried out inside an autoclave of AISI 316 steel, equipped with magnetic stirring means. To the autoclave, the reactants are charged together with the catalyst, with this latter being in an amount of 7.5 parts by weight per each 100 parts by weight of the same reactants.

The autoclave is then sealed, is dipped into a molten-salt bath, and is heated for 1 hour at 300°C (bath temperature).

At the end of the reaction, the same procedure as of Example 1 is followed, and the following results are obtained.

In Example 2, 1.09 g of hydroquinone and 4.14 g of acetamide are used; the reaction is carried out with a molar ratio of acetamide to hydroquinone of 7:1, with a catalyst of titanium-silicalite, containing silicon, aluminum and titanium in an atomic ratio to one another of 1:0.016:0.05, and a conversion, relatively to hydroquinone, of 90.8 mol % is obtained, with a selectivity to N-acetyl-para-amino-phenol of 67.5 mol %.

In Example 3, the reaction is carried out with a molar ratio of acetamide to hydroquinone of 5:1, with a catalyst of boron-silicalite, containing silicon, aluminum and boron in an atomic ratio to one another of 1:0.02:0.02, and a conversion, relatively to hydroquinone, of 70.3 mol % is obtained, with a selectivity to N-acetyl-para-amino-phenol of 63.1 mol %.

In Example 4, the reaction is carried out with a molar ratio of acetamide to hydroquinone of 1:1, with a catalyst of titanium-gallium-silicalite, in which the atomic ratio of silicon to gallium to titanium is of 1:0.01:0.018, and a conversion, relatively to hydroquinone, of 53.8 mol % is obtained, with a selectivity to N-acetyl-para-amino-phenol of 25.7 mol %.

## Claims

1. Process for preparing N-acyl-anilines characterized in that a phenol and an amide are made to react with each other by operating at a temperature within the range of from 200°C to 350°C and in the presence of a molecular sieve as the catalyst.

2. Process according to claim 1, characterized in that the N-acyl-aniline is N-acetyl-para-amino-phenol, which is obtained by means of the reaction of hydroquinone and acetamide.

3. Process according to claim 1, characterized in that the catalyst is a crystalline aluminosilicate with a high content of silica.

4. Process according to claim 3, characterized in that said catalyst is a ZSM-5 zeolite.

5. Process according to claims 1, 3 and 4, characterized in that said catalyst is in its acidic form.

6. Process according to claim 1, characterized in that the catalyst is a silicalite, which may additionally contain in its structure substituted atoms of titanium, gallium, boron and iron.

7. Process according to claim 2, characterized in that the reaction is carried out with a molar ratio of hydroquinone to acetamide comprised within the range of from 1:0.5 to 1:20.

8. Process according to claim 7, characterized in that said molar ratio is comprised within the range of from 1:1.5 to 1:10.

9. Process according to claim 2, characterized in that the reaction is carried out in the liquid phase and with an amount of catalyst comprised within the range of from 5 to 30 parts by weight per each 100 parts by weight of hydroquinone and acetamide.

10. Process according to claim 9 characterized in that said reaction temperature is of the order of 250-300°C.

## Patentansprüche

1. Verfahren zur Herstellung von N-Acyl-anilinen dadurch gekennzeichnet, daß ein Phenol und ein Amid durch Arbeiten bei einer Temperatur im Bereich von 200°C bis 350°C und in Anwesenheit eines Molekularsiebs als Katalysator miteinander zur Umsetzung gebracht werden.

2. Verfahren nach Anspruch 1, dadurch gekennzeichnet, daß das N-Acyl-anilin ein N-Acetyl-para-amino-phenol ist, das durch die Umsetzung von Hydrochinon mit Acetamid erhalten wird.

3. Verfahren nach Anspruch 1, dadurch gekennzeichnet, daß der Katalysator ein kristallines Aluminosilikat mit einem hohen Siliziumdioxidgehalt ist.

4. Verfahren nach Anspruch 3, dadurch gekennzeichnet, daß der Katalysator ein ZSM-5-Zeolith ist.

5. Verfahren nach den Anspüchen 1, 3 und 4, dadurch gekennzeichnet, daß der Katalysator in seiner sauren Form vorliegt.

6. Verfahren nach Anspruch 1, dadurch gekennzeichnet, daß der Katalysator ein Silicalit ist, der zusätzlich in seiner Struktur substituierte Atome von Titan, Gallium, Bor und Eisen enthalten kann.

7. Verfahren nach Anspruch 2, dadurch gekennzeichnet, daß die Umsetzung mit eine Molverhältnis von Hydrochinon zu Acetamid im Bereich von 1:0,5 bis 1:20 ausgeführt wird.

8. Verfahren nach Anspruch 7, dadurch gekennzeichnet, daß das Molverhältnis im Bereich von 1:1,5 bis 1:10 liegt.

9. Verfahren nach Anspruch 2, dadurch gekennzeichnet, daß die Umsetzung in flüssiger Phase und mit einer Katalysatormenge im Bereich von 5 bis 30 Gewichsteilen je 100 Gewichsteile Hydrochinon und Acetamid ausgeführt wird.

10. Verfahren nach Anspruch 9, dadurch gekennzeichnet, daß die Reaktionstemperatur in der Größenordnung von 250 bis 300°C liegt.

## Revendications

1. Procédé de préparation de N-acyl-anilines caractérisé en ce qu'on fait réagir, l'un avec l'autre un phénol et un amide, en opérant à une température de 200 à 350°C et en présence d'un tamis moléculaire utilisé comme catalyseur.

2. Procédé selon la revendication 1, caractérisé en ce que la N-acyl-aniline est le N-acétyl-para-aminophénol qui est obtenu par réaction de l'hydroquinone et de l'acétamide.

3. Procédé selon la revendication 1, caractérisé en ce que le catalyseur est un aluminosilicate cristallin ayant une teneur élevée en silice.

4. Procédé selon la revendication 3, caractérisé en ce que le catalyseur est de la zéolite ZSM-5.

5. Procédé selon les revendications 1, 3 et 4, caractérisé en ce que le catalyseur est sous sa forme acide.

6. Procédé selon la revendication 1, caractérisé en ce que le catalyseur est une silicalite, pouvant contenir en outre dans sa structure des atomes de substitution de titane, de gallium, de bore et de fer.

7. Procédé selon la revendication 2, caractérisé en ce que la réaction est mise en oeuvre avec un rapport molaire hydroquinone : acétamide compris dans l'intervalle allant de 1 : 0,5 à 1 : 20.

8. Procédé selon la revendication 7, caractérisé en ce que le rapport molaire est compris dans l'intervalle allant de 1 : 1,5 à 1 : 10.

9. Procédé selon la revendication 2, caractérisé en ce que la réaction est mise en oeuvre en phase liquide et avec une proportion de catalyseur comprise dans l'intervalle allant de 5 à 30 parties en poids pour 100 parties en poids d'hydroquinone et d'acétamide.

10. Procédé selon la revendication 9, caractérisé en ce que la température de la réaction est de l'ordre de 250 à 300°C.
